Europäisches Patentamt

(19) European Patent Office

Office européen des brevets

(11) Publication number: **0 046 669**
**B1**

(12) **EUROPEAN PATENT SPECIFICATION**

(45) Date of publication of patent specification: **24.10.84**

(21) Application number: **81303825.4**

(22) Date of filing: **21.08.81**

(51) Int. Cl.³: **C 07 C 103/52,** C 12 N 15/00, G 01 N 1/20, C 07 H 21/04, C 12 P 21/02

(54) Somatostatin or somatostatin precursors.

(30) Priority: **25.08.80 US 181046**

(43) Date of publication of application:
**03.03.82 Bulletin 82/09**

(45) Publication of the grant of the patent:
**24.10.84 Bulletin 84/43**

(84) Designated Contracting States:
**AT BE CH DE FR GB IT LI LU NL SE**

(56) References cited:
EP-A-0 001 929
EP-A-0 001 930
EP-A-0 001 931

**Chemical Abstracts vol. 88, no. 5, 30 January 1978 Columbus, Ohio, USA ITAKURA et al. "Expression in escherichia coli of a chemically synthesized gene for the hormone somatostatin" page 241, abstract no. 34407v**

(73) Proprietor: **THE REGENTS OF THE UNIVERSITY OF CALIFORNIA**
**2199 Addison Street**
**Berkeley California 94720 (US)**

(72) Inventor: **Hobart, Peter**
**2079 Golden Gate Drive**
**San Francisco California 94115 (US)**
Inventor: **Crawford, Robert**
**609 Gonzalez Drive**
**San Francisco California 94132 (US)**
Inventor: **Pictet, Raymond L., Inst. de Recherche en Biologie**
**Moleculaire Université, Tour 43, 2 Place Jussieu**
**F-75221 Paris 7, Cedex 05 (FR)**
Inventor: **Rutter, William J.**
**80 Everson Street**
**San Francisco California 94131 (US)**

(74) Representative: **De Minvielle-Devaux, Ian Benedict Peter et al**
**CARPMAELS & RANSFORD 43, Bloomsbury Square**
**London WC1A 2RA (GB)**

Courier Press, Leamington Spa, England.

(54) References cited:
Chemical Abstracts vol. 94, no. 5, 2 February 1981 Columbus, Ohio,USA, GOODMAN et al. "Nucleotide sequence of a cloned structural gene coding for a precursor of pancreatic somatostatin" page 179, abstract no. 26292n
NATURE, vol. 288, no. 5787, 13 November 1980 Chesham HOBART et al. "Cloning and sequence analysis of cDNAs encoding two distinct somatostatin precursors found in the endocrine pancreas of anglerfish" pages 137 to 141
SCIENTIFIC AMERICAN, vol. 242, no. 4, April 1980 New York GILBERT et al. "Useful proteins from recombinant bacteria" pages 68 to 82

## Description

The present invention relates to a novel somatostatin and novel somatostatin precursors, DNA transfer vectors containing a deoxynucleotide sequence coding for any one of these and microorganisms containing the transfer vector as well as a method for making the transfer vector.

Specifically, the present invention provides for a somatostatin or somatostatin precursor selected from the group comprising preprosomatostatin-1, prosomatostatin-1, preprosomatostatin-2, prosomatostatin-2 and somatostatin-2.

The invention further provides for a DNA transfer vector comprising a deoxynucleotide sequence coding for a somatostatin or somatostatin precursor selected from the group comprising preprosomatostatin-1, prosomatostatin-1, preprosomatostatin-2, prosomatostatin-2 and somatostatin-2.

In one embodiment, the DNA transfer vector contains the deoxynucleotide sequence coding for somatostatin-2.

In a further embodiment, the DNA transfer vector containing the somatostatin-2 sequence comprises a plus strand having the sequence:

5'—GCT GGC TGT AAG AAC TTC TAT TGG AAG GGC TTC ACT TCC TGT TAA—3'

wherein
    A is deoxyadenyl,
    G is deoxyguanyl,
    C is deoxycytosyl and
    T is thymidyl.

In another embodiment, the DNA transfer vector contains a deoxynucleotide coding for preprosomatostatin-1.

In a further embodiment, the DNA transfer vector containing the preprosomatostatin-1 sequence comprises a plus strand having the sequence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC GCC CAC GCC GAC·CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

wherein
    A is deoxyadenyl,
    G is deoxyguanyl,
    C is deoxycytosyl and
    T is thymidyl.

In an additional embodiment, the DNA transfer vector contains the deoxynucleotide sequence coding for preprosomatostatin-2.

In a further embodiment, the DNA transfer vector containing the preprosomatostatin-2 sequence comprises a plus strand having the sequence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

wherein
    A is deoxyadenyl,
    G is deoxyguanyl,
    C is deoxycytosyl and
    T is thymidyl.

In a still further embodiment the DNA transfer vector contains a deoxynucleotide sequence comprising a DNA strand having a deoxynucleotide sequence complementary to the ribonucleotide sequence of mRNA coding for said somatostatin or somatostatin precursor.

In a further embodiment the DNA transfer vector is pBR322/pLaS1 or pBR322/pLaS2.

The DNA transfer vectors of the invention may be used to transform microorganisms and the invention provides for microorganisms transformed by these transfer vectors.

The invention also provides for a process for preparing a DNA transfer vector containing a sequence coding for a somatostatin or a somatostatin precursor characterized by reacting said deoxynucleotide sequence with a DNA molecule prepared by cleaving a transfer vector with a restriction enzyme.

In one embodiment, a method for making the plasmid pBR322/pLaS1 or pBR322/pLaS2 is characterized in that the DNA molecule prepared by cleaving a transfer vector with a restriction enzyme is pBR322 and the restriction enzym is *Pst* I.

The invention further provides for a method for preparing an expression transfer vector characterized in that the DNA molecule prepared by cleaving a transfer vector with a restriction enzyme is cleaved at a point within an expressible control region.

The invention also provides for a method for making a fusion protein comprising the amino acid sequence of a somatostatin or a somatostatin precursor as its C-terminal end and a portion of a procaryotic protein as its N-terminal end characterized by incubating a microorganism transformed by an expression vector.

The invention further provides for a process for synthesizing a somatostatin or a somatostatin precursor selected from the group comprising preprosomatostatin-1, preprosomatostatin-2 and somatostatin-2 characterized by incubating a microorganism transformed by an expression vector comprising a deoxynucleotide sequence coding for said somatostatin or somatostatin precursor under conditions suitable for expression of said deoxynucleotide sequence and purifying said somatostatin or somatostatin precursor from the lysate or culture medium of said microorganism.

Somatostatin was originally isolated from the mammalian hypothalamus and shown to be an inhibitor of the secretion of growth hormone, Vale, W., et al., *C.R. Acad. Sci. Paris, 275,* 2913 (1972). The hormone is a peptide of 14 amino acids length. The amino acid sequence of human Somatostatin is

AlaGlyCysLysAsnPhePheTrpLysThrPheThrSerCys.

The peptide may be linear or cyclic, by disulfide bond formation between the two Cys residues, but either form is active. More recent work has shown that the hormone is more widespread throughout the body and has a broader range of regulatory activities than was previously supposed. In particular, Somatostatin has been localized in the D cells of the endocrine pancreas and has been shown to be an inhibitor of both insulin and glucagon secretion. In addition, Somatostatin has been detected in discrete epithelial cells lining the gut where it functions to inhibit the secretion of several gastrointestinal hormones including secretin, gastrin and choleocystikinin. However, Somatostatin is not universally involved in regulating the secretion of peptide hormones, since it has no effect on the secretion of follicle stimulating hormone, luteinizing hormone or thyrotropin. In addition to its regulatory role in the production of certain hormones, there is also evidence suggesting that Somatostatin functions as a neurotransmitter.

The widespread localization and breadth of this hormone's regulatory activity indicate that the somatostatin activity is of fundamental physiological significance. However, there is no evidence that the observed regulatory effects are everywhere exerted by the same compound. Indeed, peptides up to 10 times the size of Somatostatin have been found which react with Somatostatin antibodies. See Conlon, J. et al., *FEBS Letts., 94,* 327 (1978) and Noe, B. et al., *Endocrinology, 102,* 1675 (1978). One such larger peptide has been shown able to inhibit secretion of growth hormone by the pituitary. Recently, a 28 residue peptide containing the somatostatin sequence has been isolated from porcine intestinal tissue and sequenced (Pradagrol, L., *FEBS Letts., 109,* 55 (1980)).

These and other observations have prompted the suggestion that Somatostatin, in common with a number of other peptide hormones, may be synthesized as part of a large precursor peptide that is subsequently processed. Such processing could modify the known somatostatin activities, resulting in enhanced specificity or selectivity.

Analogs of Somatostatin have been chemically synthesized. Such analogs have altered relative activities in their effects on different hormones when compared with Somatostatin itself. Thus, the somatostatin analogs and peptides comprising the somatostatin sequence are known to have somatostatin activity although the relative selectivity and specificity of these activities may be different from Somatostatin itself. The magnitude of such differences may not be predictable from knowledge of the structure alone. As a general principle, it would be expected that larger proteins comprising the somatostatin sequence display more specificity of activity. In fact, the emerging picture is that Somatostatin itself is but one of a family of related peptides, all of whom function to inhibit the secretion of peptides, especially peptide hormones, but which may differ in amino acid sequence, depending upon their source and target tissue. We refer herein to this general class of peptides as somatostatins.

Because of their known biological activities, the somatostatins have utility for the regulation of hormone output and for the treatment of disease conditions characterized by hormone overproduction. Somatostatin is subject to rapid degradation by proteases. In addition, Somatostatin itself is rapidly excreted following parenteral administration, making it difficult to control the effective dose. Larger peptides are excreted more slowly. Therefore, higher molecular weight somatostatins provide the means for more readily obtaining and controlling a satisfactory effective dose. The somatostatins will be advantageous for the treatment of a broad range of hormone imbalance conditions. For example, a somatostatin selectively inhibiting glucagon but not insulin secretion would be beneficial for insulin-dependent diabetics who suffer ill effects from relative overproduction of glucagon, which exacerbates hyperglycemia. Also, proliferative retinopathy, which is a frequent pathology in diabetes, is linked to the overproduction of growth hormone in such patients and therefore adult patients can benefit from administration of a somatostatin. Constant maximal secretion induced by hyperglycemia may be deleterious to B cells. Some individuals produce a small but insufficient quantity of endogeneous insulin at maximum capacity. These patients can benefit from administration of an exogenous somatostatin. Patients whose glucagon synthesis is relatively normal would benefit similarly from administration of a somatostatin lacking the activity of inhibiting glucagon secretion.

Somatostatin has been purified from various tissue sources and has also been synthesized by ordinary peptide chemistry techniques. In addition, Somatostatin has been synthesized by a microorganism using recombinant DNA techniques, Itakura, K. et al., *Science, 198,* 1056 (1977), EP—A—0001929, EP—A—0001930 and EP—A—0001931. In these instances, the DNA coding for the amino acid sequence of Somatostatin was chemically synthesized on the basis of the known amino acid sequence, and the coding relationships of the genetic code. The codons were selected on the basis of supposed compatibility with the host microorganism, E. coli. Until the present invention, we believe that the naturally-occurring DNA sequence coding for somatostatin has never been isolated and characterized. Consequently, no information regarding precursor proteins was available.

Summary of the invention

The present invention discloses the cloning of cDNA containing naturally occurring sequences coding for somatostatins comprising both known and novel somatostatin sequences. For the purpose of this application, the following nomenclature is employed:

Somatostatin 1 (S1) refers to the 14 amino acid peptide heretofore termed Somatostatin, *supra*, and in the prior art.

Somatostatin 2 (S2) is a novel, naturally occurring somatostatin.

Preprosomatostatin 1 (Prepro S1) is a novel protein comprising S1 as its C-terminal sequence, and having as its N-terminal sequence a sequence of hydrophobic amino acids characteristic of the signal peptide described for other precursors of secretory proteins, Devillers-Thiery, A. et al., *Proc. Nat. Acad. Sci. USA, 72,* 5016 (1975).

Prosomatostatin 1 (Pro S1) results from the removal of the signal peptide from Prepro S1.

Similarly, preprosomatostatin 2 (Prepro S2) and prosomatostatin 2 (Pro S2) are novel proteins comprising the S2 amino acid sequence at the carboxyl terminus and a signal peptide in the case of prepro S2 or lacking the signal peptide in the case of pro S2. Prepro S1 has a molecular weight approximately the size of the "big somatostatin" molecule which has been detected in both mammalian and fish tissue, Conlon, J. et al., *supra,* and Noe, B et al., *supra.*

Prepro S1 and prepro S2 are considered to be naturally occurring precursor molecules from which active somatostatins, including pro S1, S1, pro S2 and S2 are derived by additional processing steps.

The present invention provides for the synthesis of the foregoing novel somatostatins in a microorganism transformed by cDNA coding appropriately for the desired protein, said cDNA being inserted in a transfer vector suitable for obtaining expression of the cloned insert, either as a protein product or as a fusion protein coupled to a segment of bacterial protein. The present invention therefore includes the novel somatostatins per se, new methods of making both the novel somatostatins and S1, transfer vectors comprising inserted coding sequences for the foregoing somatostatins, new microorganisms transformed by the above-mentioned transfer vectors, and new fusion proteins comprising the amino acid sequences of the foregoing somatostatins.

Detailed description of the invention

Since a naturally occurring coding sequence comprising the coding region for S1 had never previously been isolated, it was necessary to use as starting material a tissue known to have a sufficiently high proportion of somatostatin-producing cells to permit the practical isolation of mRNA sufficiently enriched in somatostatin coding sequences. In the anglerfish (*Lophius americanus*), the endocrine pancreas, termed Brockmann body, is easily separable from exocrine tissue and is highly enriched in D cell types (somatostatin-producing) relative to B cells (insulin-producing). Furthermore, the amino acid sequence of S1 isolated from anglerfish Brockmann body cells is identical to mammalian S1, Noe, B. et al., *Endocrinology, 105,* 1410 (1979).

A variety of cDNA inserts were synthesized from Brockmann body mRNA and cloned. The clones were then tested for their ability to cross-hybridize with each other. In this manner, it was determined that the majority of cDNA clones fell into one of three groups.

Subsequent analysis showed that these groups included cDNA coding for fish preproinsulin, prepro S1 and, unexpectedly, the sequence coding for prepro S2. The techniques of the present invention have made extensive use of enzyme-catalyzed reactions.

RNA is isolated from Brockmann body cells using conventional techniques. Polyadenylated RNA is isolated by affinity chromatography. The polyadenylated RNA is used as the template for preparing a double stranded cDNA copy using conventional techniques. The first cDNA strand is synthesized using reverse transcriptase, an olig-dT primer and the polyadenylated RNA. After synthesis of the first strand of cDNA, the RNA is removed by alkali digestion and the single-stranded cDNA is used to self-prime the synthesis of the second strand by reverse transcriptase. The single-stranded hairpin loop is removed by digestion with S1 nuclease.

The cDNA is now ready for insertion into an appropriate transfer vector. Suitable transfer vectors include those which are capable of transforming bacteria, such as *Escherichia coli* and *Bacillus subtilis,* yeast (*Saccharomyces cerevisiae*), the inl, csp and esp mutant strains of *Neurospora crassa* and animal cells in tissue culture. Examples of transfer vectors suitable for use in this invention which are capable of transforming *E. coli* include: the plasmids pSC101, pMB9, pBR313, pBR315, pBR316 and pBR322 and the vectors derived from bacteriophage, i.e., Charon 3A, Charon 4A, Charon 16A and *λ*gtWES · *λ*B. References which describe the preparation and characterizations of these transfer vectors are listed in Table 1.

TABLE 1

| Transfer vector | Reference(s) |
| --- | --- |
| pSC101 | Cohen, et al., *Proc. Natl. Acad. Sci. USA, 70,* 1293 and 3240 (19—7). |
| | Boyer, et al., *Recombinant Molecules,* Beers, et al., Eds., Raven Press, NY, at p. 13 (1977). |
| | Cohen, et al., *Recombinant Molecules,* at p. 91. |
| pMB9 | Rodriguez, et al., *Molecular Mechanisms in Control of Gene Expression* (ICN-UCLA Symposium on Mol. & Cell. Biol. Vol. 5), Nieslich, et al., Eds., Academic Press, NY, at p. 471 (1976). |
| | Bolivar, et al., *Gene, 2,* 75 (1977). |
| pBR313 | Bolivar et al., *supra.* |
| pBR315 | Rodriguez et al., *supra.* |
| pBR316 | Rodriguez et al., *supra.* |
| pBR322 | Bolivar et al., *Gene, 2,* 95 (1977). |
| | Bolivar et al., *Gene, 4,* 121 (1978). |
| | Sutcliffe, *Cold Spring Harbor Symp. on Quant. Biol., 43,* 77 (1978). |
| Charon 3A | Blattner et al., *Science, 196,* 161 (1977). |
| Charon 4A | Blattner et al., *supra.* |
| Charon 16A | Blattner et al., *supra.* |
| *λ*gtWES · *λ*B | Tremeier et al., *Nature, 263,* 526 (1976). |
| *λ*gtWES · *λ*B | Leder et al., *Science, 196,* 175 (1977). |

Other suitable vectors for transforming *E. coli* are described in Sinsheimer, R. L., *Ann. Rev. Biochem., 46,* 415 (1977). Suitable transfer vectors for transforming *B. subtilis* have been described by *Iordanescu,* S., *J. Bacteriol., 124,* 597 (1964) and Ehrlich, S. D., *Proc. Natl. Acad. Sci. USA, 74,* 1680 (1977). One such vector has been identified as plasmid pC194. Hybrid plasmids containing yeast DNA—either plasmid and/or chromosomal DNA—and bacterial DNA—generally *E. coli* plasmid DNA—are used to transform yeast. Such plasmids have been described by Beggs, J. D., *Nature 275,* 104 (1978); Asiav, C. L. and Carbon, J., *Proc. Natl. Acad. Sci. USA 76,* 3829 (1979); and Kingsman, A. K., et al., *Gene 7,* 141 (1979). Transfer vectors which can be utilized to transform animal cells in tissue culture include Adeno defective virus, SV-40 defective virus and polyoma virus.

The cDNA is inserted into a transfer vector using conventional techniques. The cDNA is usually inserted into any restriction site which is unique in the transfer vector. Charon 3A and 4A and *λ*gtWES · *λ*B do not contain unique sites and, as a result, restriction sites of limited number are used for these vectors. The unique restriction sites in various transfer vectors and the useful restriction sites in vectors Charon 3A and 4A and *λ*gtWES · *λ*B are shown in Table 2.

## TABLE 2

| Transfer vector | Unique restriction sites |
| --- | --- |
| pSC101 | *Eco*Ri, *Hin*d III, *Sal* I, *Bam* HI, *Hpa* I, *Sma* I |
| pMB9 | *Eco*Ri, *Hin*d III, *Sal* I, *Bam* HI |
| pBR313 | *Eco*RI, *Hin*d III, *Sal* I, *Bam* HI, *Hpa* I, *Sma* I, *Xma* I |
| pBR315 | *Eco*RI, *Hin*d III, *Sal* I, *Bam* HI, *Pst* I |
| pBR316 | *Hin*d III, *Sal* I, *Bam* HI, *Pst* I |
| pBR322 | *Eco*RI, *Hin*d III, *Sal* I, *Bam* HI, *Pst* I |
| pC194 | *Hin*d III |
| Charon 16A | *Eco*RI, *Sst* I |
| Charon 3A | *Eco*RI |
| Charon 4A | *Eco*RI |
| λgtWES · λB | *Eco*RI |

Generally, the cDNA is inserted into a restriction site within a phenotypic trait for ease in selection. For example, *Hin*d III, *Sal* I and *Bam* HI in pSC101, pMB9, pBR313, pBR315, pBR316, and pBR322 are within the gene for tetracycline resistance or its control region. Insertion at this site results in loss of tetracycline resistance. The cDNA can be inserted into the transfer vector by the use of restriction site linkers or deoxynucleotide tailing. In the former, a synthetic nucleotide containing a recognition site for a particular restriction endonuclease, such as those discussed above, can be blunt-end ligated to the cDNA. The cDNA and transfer vector are separately incubated with the restriction endonuclease and then annealed to form the transfer vector containing the cDNA. In the latter, the cDNA can be tailed using a deoxynucleotide and terminal transferase as described by Roychoudhury, R., et al., *Nucl. Acids Res., 3,* 863 (1976). The transfer vector, after digestion with a restriction endonuclease, can be tailed using the complementary deoxynucleotide in the same procedure. The tailed transfer vector and tailed cDNA are then annealed to form the transfer vector containing the cDNA. For example, the cDNA can be dC-tailed and the transfer vector can be opened at the *Pst* I site and dG-tailed.

The transfer vector containing the cDNA is then used to transform a suitable host. Hosts which are suitable for the various transfer vectors include *E. coli, B. subtilis,* yeast, *N. crassa,* and animal cells in tissue culture. The transfer vectors which are capable of transforming each of these hosts have been described above. Three mutant strains of *E. coli* are conventionally utilized. These are $\chi$1776, RRI and HB101. *E. coli* $\chi$1776 has been described in Curtis, R., *Ann. Rev. Microbiol. 30,* 507 (1976) and U.S. Patent No. 4,190,495. *E. coli* RRI has been described in Dugaiczyk, A., et al., *Molecular Mechanisms in Control of Gene Expression* at p. 543. *E. coli* HB101 has been described in Kedes, D. H. and Thomas, C. A., *Proc. Natl. Acad. Sci. USA 73,* 1537 (1976). The suitable hosts are transformed by conventional techniques. For example, *E. coli* $\chi$1776 is transformed by the transfer vector containing the cDNA as described by Cooke, N. E., et al., *J. Biol. Chem., 255,* 6502 (1980). Colonies are selected and/or screened by conventional techniques. For example, colonies may be selected based on the loss of a phenotypic trait, such as tetracycline resistance. Colonies may be screened by (1) removing the cDNA by an appropriate restriction endonuclease and analyzing it by electrophoresis and hybridization (Southern, E. M., *J. Mol. Biol. 98,* 503 (1975)), or (2) replicating as described by Grunstein, M. and Hogness, D. S., *Proc. Natl. Acad. Sci. USA 72,* 3961 (1975) and hybridizing with an appropriate problem, or (3) examining colonies directly for expression by RIA or other techniques.

The cloned DNAs designated pLaS1 and pLaS2 are expressed in bacteria to yield either fusion proteins comprising the peptides coded by the inserted sequences, or the peptides themselves. Several possible techniques are available as options, and may include (a) modification of the coding sequences to provide an exact desired translational starting point; (b) selection or construction of an optimal expression vector; and (c) post-translational processing, either by exploiting *in vivo* processing activity of the host or by *in vitro* chemical means.

Where a fusion protein is expressed, modification of the cloned nucleotide sequence will generally be unnecessary as long as the resulting sequence permits translation of the insert in the correct reading frame and no stop codons intervene before the initial codon of the inserted sequence. Both S1 and S2 may be expressed conveniently as fusion proteins followed by post-translational *in vitro* processing, since both are joined to the rest of the Pro S1 or Pro S2 sequences by the dipeptide arg-lys. Such a sequence is especially susceptible to tryptic hydrolysis. Both S1 and S2 are readily released by partial tryptic digestion of their respective precursors.

Prepro S1 and Prepro S2 are expressed as fusion proteins by insertion into appropriate sites within expressed operons including, for example, the *Pst* I site in the β-lactamase gene of pBR322 (Villa-Komaroff, et al., *Proc. Nat. Acad. Sci. USA, 75*, 3727 (1978)), the *Eco*RI site of pBR322 carrying the lac control region and coding sequence for β-galactosidase (Itakura, K., et al., *Science, 198*, 1056 (1979)), or the *Hin*d III site of the trpD gene of plasmid p*trp*ED50 (Martial, J., et al., *Science, 205*, 602 (1979)). Modifications of sequence length by one or two nucleotides in order to achieve correct reading frame phase are well known in the art. Insertions at the *Pst* I site of pBR322, with the aid of the tailing procedure, occur in correct phase and reading frame with a probability of 1/6.

Pro S1 and Pro S2 are prepared from fusion proteins susceptible of specific cleavage *in vitro*. The cloned nucleotide sequences are modified to code for amino acid sequences providing specificity for a proteolytic enzyme. A useful sequence is

AspAspAspAspLys,

cleaved preferentially by the enzyme enterokinase, as described in copending application Ser. No. 125,878, filed February 29, 1980, incorporated herein by reference. As described therein, a linking nucleotide sequence coding for the foregoing amino acid sequence is inserted adjacent the nucleotide sequence coding for the desired amino terminus of Pro S1 or Pro S2.

Such insertion requires modification of pLaS1 or pLaS2, by removal of nucleotides on the 3′ end of the Pro S1 or Pro S2 coding sequence. This will be accomplished either by controlled digestion of the 3′ end of the insert using 3′ endonuclease or T4 DNA polymerase, or by the combination of restriiction endonuclease cleavage at a point to the 5′ side of the desired starting point and chemical synthesis to restore that portion of the desired sequence thus removed. Controlled digestion is preferably carried out using T4 polymerase, which, in the absence of added deoxynucleotide triphosphates, catalyzes 3′ to 5′ exonucleolytic digestion of double-stranded DNA (Englund, P. T., *J. Biol. Chem., 246*, 3269 (1971)). The extent of digestion is controlled by selection of proper temperature, reaction time and amount of enzyme, according to principles well known in the art. Experimentation will be necessary in each instance, since optimal reaction conditions must be determined for each lot of enzyme and for each DNA to be modified. By these means, the extent of digestion can be controlled. Termination of digestion at a predetermined stopping point is achieved by including a single deoxynucleotide triphosphate in the reaction mixture, corresponding to the desired stopping point. For example, in the presence of dATP, the DNA is digested 3′—5′ until the polymerase reaches a dA residue, at which point further net digestion ceases. Several cycles of digestion, each with its predetermined stopping point, can be carried out in sequence, to construct DNA molecules having a predetermined end point. Exonucleolytic digestion with T4 polymerase affects only the strands having 3′ termini. The complementary strands remain as unpaired single-stranded tails, which must also be removed. S1 nuclease is the preferred enzyme for the purpose. The product of combined treatment with T4 polymerase and S1 nuclease is blunt-ended, double-stranded DNA. Chemical synthesis of deoxynucleotide sequences used to replace segments cleaved by restriction endonuclease digestion is carried out, for example, as described by Itakura, K., et al., *J. Biol. Chem., 250*, 4592 (1975) and Itakura, K., et al., *J. Am. Chem. Soc., 97*, 7327 (1975).

Prepro S1 and Prepro S2 are synthesized in similar fashion, expressed initially as fusion proteins.

By the use of appropriate expression transfer vectors, the somatostatins of the present invention are also expressed directly, i.e., not fused to any procaryotic protein. In general, it is necessary to modify the cloned inserts, pLaS1 and pLaS2, to remove the 5′ untranslated region preceding the desired starting point. Where Prepro S1 and Prepro S2 are the primary expression products, the other somatostatins are obtained from them.

The underlying principle of direct expression is that the inserted DNA segment entirely replaces the coding segment normally transcribed and translated by the bacterial control region. The essential component of the control region to be preserved is termed the expression unit. The expression unit includes a promoter and a ribosomal binding site capable of acting in the host organism. As a practical matter, it is not necessary to remove precisely the nucleotides coding for the host portion of the fusion protein. The relationship between the ribosomal binding site and the start codon (AUG) is such that the start codon may be located anywhere within 3 to 11 nucleotides of the ribosomal binding site, Shine et al., *Proc. nat. Acad.*

*Sci. USA*, *71*, 1342 (1974); Steitz, J., et al., *Proc. Nat. Acad. Sci USA*, *72*, 4734 (1975). In this 3-11 nucleotide region, the first AUG to be encountered sets the reading frame for translation. In the case of p*trp*E30, derived from p*trp*ED50 described *supra* and containing the operator, promoter, attenuator and ribosome binding sequences of the tryptophan operon together with the nucleotide sequence coding for seven amino acids of the *trp*E protein followed by a *Hin*d III site, the removal of a minimum of 23—29 nucleotides from the *Hin*d III site provides a site for insertion into an expression unit under tryptophan operon control.

For the direct expression of Prepro S1, for example, the original cDNA insert is modified as described above to remove the 5' untranslated region. A vector for direct expression can be constructed by modification of p*trp*E30 by removing 23—29 nucleotides using T4 DNA polymerase and S1 nuclease as described above. A linker nucleotide sequence containing the restriction sequence for *Bam* HI endonuclease is blunt-end ligated to both the modified cDNA insert and the modified p*trp*E30 by the procedure of Valenzuela, et al., *supra.* This is done to facilitate insertion which is performed essentially as described by Ullrich, A., et al., *Science, 196,* 1313 (1977). Suitable hosts, such as *E. coli* HB101, RRI or χ1776 or other bacteria are transformed by the recombinant vectors bearing the inserted Prepro S1 coding region. Transformants are selected for resistance to ampicillin and then grown under conditions suitable for expression of Prepro S1.

Prepro S1 and Prepro S2 are converted to Pro S1 and Pro S2, respectively, by removal of the N-terminal sequence of hydrophobic amino acids that comprise the signal peptide. *In vitro* removal of the signal peptide is carried out by treating the protein extracted from transformed, induced cells, with a preparation of "rough" microsomes, as described by Jackson, R. C., Blobel, G. (1977), *Proc. Nat. Acad. Sci. USA, 74,* 5598. *In vivo* removal of the signal peptide may occur during direct bacterial expression of the Prepro S1 or Prepro S2 coding sequence. Bacterial and mammalian signal peptides share sequence similarities. Proteins having mammalian signal peptides may be processed by bacterial cells, resulting in excretion of the propeptide (in this instance Pro S1) into the periplasmic space or into the medium. This occurrence of such bacterial processing is detectable by observing the occurrence of a protein in the medium or periplasmic space of induced cells, not found in uninduced cells, as measured by incorporation of a radioactive amino acid or by radioimmunoassay.

S1 and S2 themselves may be prepared by tryptic digestion of either Prepro S1 or Pro S1 and Prepro S2 or Pro S2, respectively, as described *supra*. Either whole cell extracts or partially purified protein preparations may be treated in this fashion to yield S1 or S2.

Prepro S1, Pro S1, S1, Prepro S2, Pro S2 and S2, synthesized as described, are purified by techniques well known in the art, including, for example, gel filtration, ion exchange chromatography, affinity chromatography and differential solubility techniques. The amino acid sequences are confirmed by conventional methods of amino acid sequence analysis.

Purified enzymes for use in the practice of the present invention are presently available from commercial sources. Restriction endonucleases, their nomenclature and site specificity have been described in detail by Roberts, R., *Crit. Rev. Biochem., 4,* 123 (1976). The restriction enzymes used in this work were obtained from New England BioLabs, Beverly, Massachusetts, and used in amounts and under reaction conditions specified by the manufacturer for each enzyme. Reverse transcriptase was provided by Dr. J. Beard, Life Sciences, Inc., St. Petersburg, Florida. DNA polymerase I and T4 polynucleotide kinase were obtained from New England BioLabs, Beverly, Massachusetts. Micrococcal S1 nuclease was obtained from Miles Laboratories, Elkhart, Indiana. Bacterial alkaline phosphatase was obtained from Worthington Biochemical Corporation, Free-hold, New Jersey.

Example 1

*Synthesis of cDNA.* RNA was extracted from anglerfish Brockmann bodies obtained from Biological Associates, Portland, Maine, by the guanidine thiocyanate method, Chirgwin et al., *Biochemistry, 24,* 5194 (1979). One gram of Brockmann body tissue yields approximately 6.5 mg of total RNA. Polyadenylated RNA was purified using oligo-dT-cellulose affinity chromatography, Aviv, H. et al., *Proc. Nat. Acad. Sci. USA, 69,* 1409 (1972). 1.3 $\mu$g of single-stranded cDNA was made from 10 $\mu$g polyadenylated RNA using reverse transcriptase (Lot No. 1577) in the following reaction mix of 200 $\mu$L volume: 40 mM tris-HCl, pH 8.3, 10 mM MgCl$_2$, 56 mM $\beta$-mercaptoethanol, 0.5 mM dATP, dGTP, dTTP, 0.15 mM $^3$H-dCTP (sp. Act. 5.2 mCi/$\mu$g/ml polyadenylated RNA, 15 units reverse transcriptase $\mu$g RNA. Incubation was at 40°C for 30 minutes and then at 68°C for two minutes. The reaction was stopped by the addition of NaEDTA (pH 8.0) to 20 mM and the reaction mixture was then chromatographed over G-75 Sephadex (trademark Pharmacia, Inc., Uppsala, Sweden). The DNA-containing fraction was ethanol precipitated. RNA was removed by alkali treatment (0.2M NaOH, 23 min. at 68°C) and the second strand cDNA was carried out in the following reaction mix using DNA polymerase I: 100 mM Na HEPES pH 6.9, 75 mM KCl, 10 mM MgCl$_2$, 10 mM dithiothreitol (DTT), 100 mM each of the four deoxynucleotide triphosphates, 0.8 $\mu$g/ml single-

stranded cDNA (boiled three minutes just prior to addition), and 75 units E. coli DNA polymerase I/µg DNA. Incubation was at 14°C for two hours. The reaction volume was made 20 mM NaEDTA, extracted once with phenol-chloroformisoamyl alcohol (50:48:2 by volume) and passed over a G-75 Sephadex column. In preparation for dC tailing, the hairpin loops of the double-stranded DNA (ds-cDNA) were clipped and made blunt-ended using S1 microccocal nuclease. S1 digestion was carried out at 37°C for 30 minutes in 100 µl reaction volume, containing 0.3M NaCl, 0.03M NaOAc, 3 mM $ZnCl_2$, pH 4.5, 175 S1 units/µg ds-cDNA. After organic extraction and G-75 Sephadex chromatography to remove small DNA fragments, 300 ng of trimmed ds-cDNA was tailed using calf thymus terminal transferase in a 30-minute reaction at 37°C containing 140 mM potassium cacodylate, 30 mM tris base (pH 7.8), 1 mM $CoCl_2$, 0.1 mM DTT, 25 M dCTP ($\alpha^{32}$P-dCTP, 8.36 µCi/nmole), and 150 units terminal transferase/µg ds-cDNA. The tailing reaction is described, generally, by Roychoudhury, R., et al., Nucl. Acids Res., 3, 101 (1976). Preliminary experiments indicated approximately 30 bases are added to the 3' end under these conditions. Plasmid pBR322 was cleaved by Pst I endonuclease and provided with dG tails by the previously described tailing reaction, except that dGTP was used instead of dCTP, and no radioactive label was employed. Equimolar amounts of dC tailed cDNA and dG tailed pBR322 were annealed at a concentration of 1 µg/ml, using sequential two-hour incubations at 42°C, 30°C and 14°C (36). The hybrid plasmid DNA was ethanol precipitated and then used to transform E. coli χ1776 and selected for tetracycline resistance. To screen with various labeled probes, recombinant colonies were grown on Whatman 541 cellulose paper, lysed in situ and prepared for hybridization according to the procedure of Grunstein et al. and Proc. Nat. Acad. Sci. USA, 72, 3961 (1975). The filters were hybridized to a $^{32}$P labeled cDNA probe synthesized from anglerfish Brockmann body polyA+RNA, and plasmid DNA was isolated from those colonies giving a strong autoradiographic signal using a procedure described by Staudenbauer, W. L., Mol. Gen. Genet., 145, 273 (1976). Several plasmids were selected on the basis of their size as measured by gel electrophoresis and their cDNA inserts were purified on preparative acrylamide gel electrophoresis. Purified insert cDNA from these plasmids was labeled by nick translation and hybridized with other purified cDNAs in pair-wise combinations. By this means the inserts could be divided into a number of non-cross-hybridizing groups and their relative frequency estimated. The most frequently occurring insert among the colonies selected as described was shown by nucleotide sequence analysis to code for fish preproinsulin. Two additional groups were analyzed and specific inserts, designated pLaS1 and pLaS2, were selected for analysis.

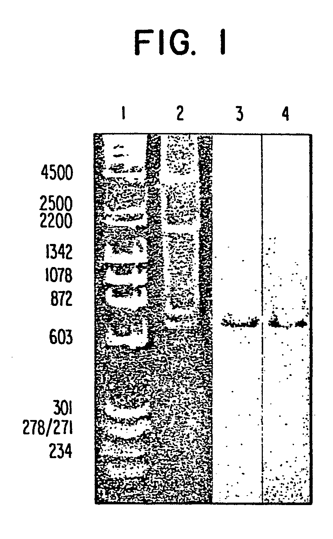

The two selected inserts were analyzed to estimate the size of the corresponding mRNA specific to each, by the method of Alwine et al., Proc. Nat. Acad. Sci. USA, 74, 5350 (1977). The method entailed hybridization to anglerfish polyadenylated RNA which had been previously chromatographed on a 2% agarose methyl mercury gel (Bailey, J., et al., Anal. Biochem., 70, 75 (1976)), and transferred to diazotized cellulose paper (Alwine, Jr., et al., supra). Hybridization was for 48 hours at 42°C. Hybridized fibers were autoradiographed after washing three times (100 ml volume, 10 minutes each) in 0.3 M NaCl, 0.03 M NaCitrate, 0.1% (w/v) SDS at 20°C and then in the same manner using 5 mM NaCl, 1.5 mM NaCitrate 0.1% SDS at 50°C. The results are shown in Figure 1. Lane 1: Hind III digested λDNA and Hae III digested φX174 DNA molecular·weight standards; Lane 2: Ethidium bromide staining of anglerfish Brockmann body (1 µg polyadenylated RNA); Lane 3: Hybridization of transferred anglerfish polyA+RNA to pLaS1 $^{32}$P insert DNA; Lane 4: Hybridization of transferred anglerfish polyA+RNA to pLaS2 $^{32}$P insert DNA.

From the data of Figure 1, it can be seen that both pLaS1 and pLaS2 hybridize specifically to mRNAs of approximately 700 bases, corresponding to the size of one major class of polyadenylated mRNA found in the Brockmann body. By a similar analysis, the other major polyadenylated mRNA of 840 bases was shown to correspond to inserts coding for preproinsulin.

Example 2

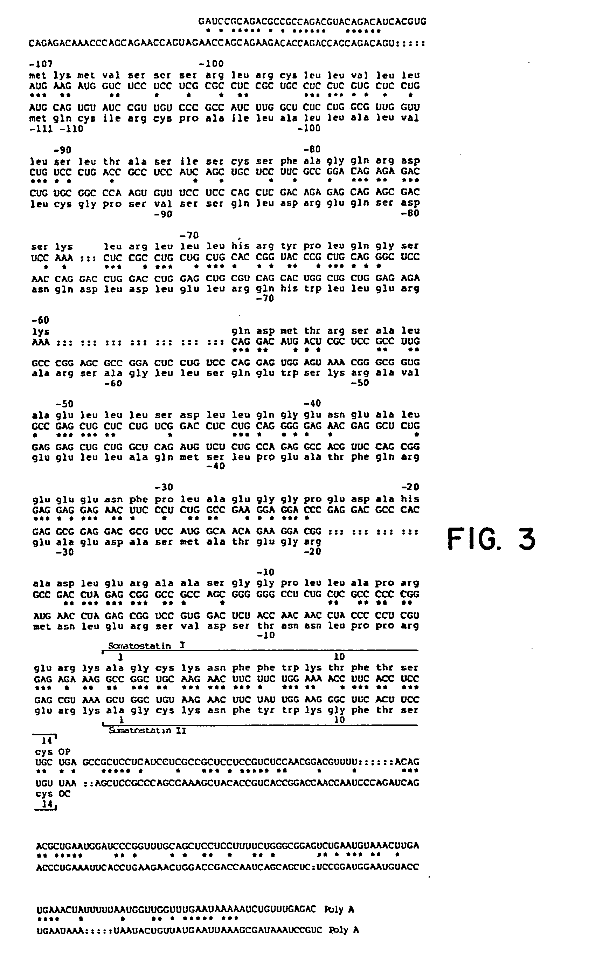

The deoxynucleotide sequences of pLaS1 and pLaS2 were analyzed by the method of Maxam, A. et al., Proc. Nat. Acad. Sci. USA, 74, 560 (1977). The ribonucleotide sequences are shown in Figure 3, together with the corresponding predicted amino acid sequences coded by the "sense" strands of the DNA, i.e., the strands corresponding in sequence to the respective mRNAs. The deoxynucleotide sequence of the sense strand is identical except that T replaces U. The correct reading frame is recognized by a lack of termination codons over a substantial portion of the insert and, in the case of pLaS1, by the fact that it correctly codes for S1. The amino acid positions are numbered beginning with the amino-terminal amino acid of S1 and proceeding, in the positive direction, to the carboxy terminal end of S1, and in the negative direction to the first AUG codon (position—107), presumed to be the point of translation initiation.

Beginning with the first AUG codon at the 5' end of the sense strand, translation of pLaS1 predicts the sequence of a 121 amino acid peptide which contains the S1 amino acid sequence at its carboxy terminus. The 121 amino acid peptide is a heretofore undescribed somatostatin, termed prepro S1 herein, having a molecular weight of 13,328.

Our results suggest that, in common with many other hormones, the synthesis of S1 involved post-translational processing. The translation of somatostatin mRNA yields a precursor (prepro S1) containing a signal peptide which may be released during the transit into the endoplasmic reticulum, and the resultant precursor (pro S1) is subsequently cleaved to yield S1 itself. By analogy with other signal peptides, and in particular with that of anglerfish insulin with which it shares sequence homology, the prepeptide portion of prepro S1 is probably about 20—25 bases long. After removal of the signal peptide, the resultant pro S1 molecule would have approximately 96—101 amino acid residues and a molecular weight of approximately 9,000. Pro S1 is more than five times the size of somatostatin.

The pLaS2 deoxynucleotide sequence was analyzed in a manner similar to that described for pLaS1. Translation of pLaS2 predicts the sequence of a 125 amino acid peptide which surprisingly contains a 14 amino acid sequence at its carboxy terminus which differs from S1 by only two amino acids, and is termed Somatostatin 2 (S2) herein. Three novel somatostatins are therefore contained within the full sequence: S2, Pro S2, and Prepro S2. The predicted molecular weights of Prepro S2 and Pro S2 are 14,100 and 11,600, respectively. Figure 3 shows the corresponding ribonucleotide and amino acid sequences of pLaS2. The deoxynucleotide sequence is identical except that T replaces U.

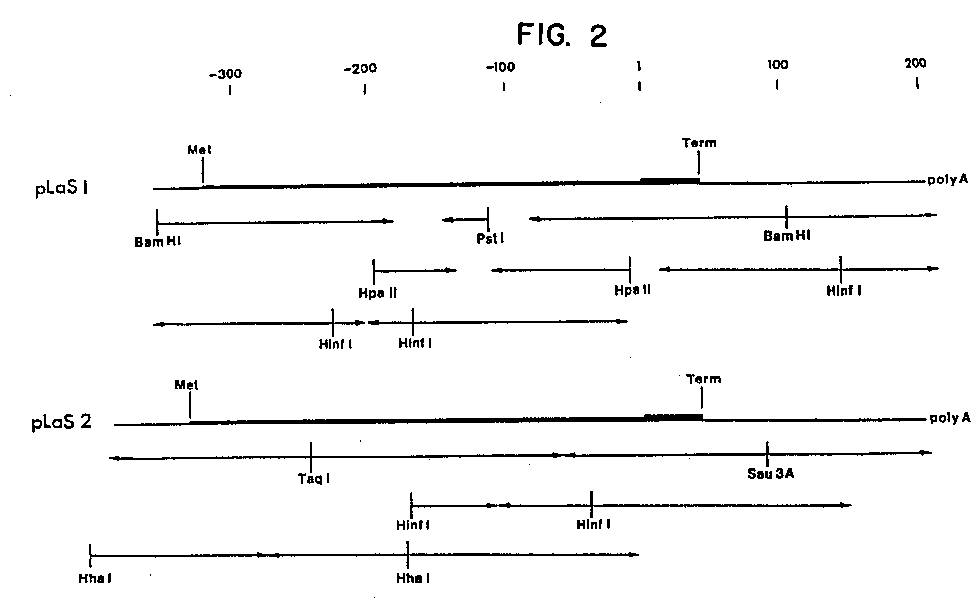

Figure 2 shows hybridized transfer vectors containing the S1 and S2 sequences in pLaS1 and pLaS2 respectively, with particular restriction sites identified.

Example 3

(A) Example 1 is repeated using plasmid pBR315 in place of plasmid pBR322. All conditions including selection of recombinant clones are as described. The inserts are removed and analyzed as described in Example 2, yielding DNA's of about 700 base pairs having the sequences shown in Figure 3.

(B) Example 1 is repeated using plasmid pBR316 in place of plasmid pBR322. All conditions are identical to those described in Example 1. Removal and analysis of the inserts as described in Example 2 yields DNA's having the sequences shown in Figure 3.

Example 4

For this example, the cDNA is prepared as described in Example 1. All restriction site linkers utilized in this example and those that follow are prepared as described by Scheller, R. H. et al., *Science 196,* 177 (1977). The restriction endonucleases used in this Example and those that follow are commercially available from New England BioLabs, Beverly, Massachusetts, and optical conditions according to supplier's recommendations are utilized. Transformation of *E. coli* $\chi$1776 is performed as described in Example 1.

(A) *Hin*d III linkers having the sequence 5'—CCAAGCTTGG—3' are ligated to the cDNA prepared in Example 1 by blunt-end ligation using T4 DNA ligase as described by Valenzuela, P., et al., *Nature 280,* 815 (1979). After ligation, the product is digested with *Hin*d III or *Hsu* I following the procedure described by Ullrich, A. et al., *Science 196,* 1313 (1977). Plasmid pBR322 is cleaved at the *Hin*d III restriction site with either *Hin*d III or *Hsu* I and treated with alkaline phosphatase as described by Ullrich, A., et al., *supra*. After ethanol precipitation, the phosphatase-treated, cleaved pBR322 is ligated to the cDNA containing *Hin*d III cohesive termini as described by Ullrich, A., et al., *supra. E. coli* $\chi$1776 is transformed with the ligation mixture and the transformed colonies are selected for tetracycline sensitivity. The presence of cloned DNA is determined as described in Example 1. Colonies containing inserts are selected as described in Example 1. The inserts are removed by digestion with *Hin*d III or *Hsu* I and analyzed as described in Example 2. The inserts contain about 700 base pairs and have the sequences shown in Figure 3.

(B) Example 4(A) is repeated in which several vectors are utilized in place of pBR322. In this Example, the plasmids pSC101, pMB9, pBR313, pBR315, and pBR316 are utilized in place of pBR322. All conditions including selection of recombinant clones are as described in (A). For all plasmids, identical results are obtained, i.e., recombinant plasmids containing inserts having the sequences shown in Figure 3 are obtained in each instance.

(C) Example 4(A) is repeated in which several other restriction site linkers and restriction endonucleases are utilized. In this Example, the linkers for *Sal* I and *Bam* HI are used in place of the linkers for *Hin*d III. The sequences of these linkers are

$$5'—GGTCGACC—3'$$
$$\text{and}$$
$$5'—CCGGATCCGG—3'.$$

When *Sal* I linkers are used, the linker-treated cDNA and pBR322 are cleaved with restriction endonuclease *Sal* I and when *Bam* HI linkers are utilized, the restriction endonuclease is *Bam* HI. All conditions including selection of recombinant clones are as described in 4(A). For both linkers and restriction endonucleases, identical results are obtained.

(D) Example 4(C) is repeated in which plasmids pSC101, pMB9, pBR313, pBR315 and pBR316 are used in place of pBR322. All conditions are the same as described in Example 4(C) and identical results are obtained, i.e., in each instance inserts having the sequences shown in Figure 3 are obtained.

(E) Example 4(A) is repeated in which an *Eco* RI restriction linker having the sequence 5'—CCGAATTCGG—3' is used in place of the *Hin*d III linker and *Eco* RI is used in place of *Hin*d III (*Hsu* I). All conditions are identical to those described except that transformed colonies are not selected by tetracycline sensitivity. The presence of cloned DNA is performed as described in Example 1. Recombinant clones containing inserts having the sequences shown in Figure 3 are obtained.

(F) Example 4(E) is repeated in which plasmids pSC101, pMB9, pBR313 and pBR315 are used in place of pBR322. The same conditions as described in Example 4(E) are utilized and identical results are obtained for each plasmid.

(G) Example 4(A) is repeated in which a *Pst* I restriction linker having the sequence

$$5'—GCTGCAGC—3'$$

and *Pst* I are used in place of the *Hin*d III linker and *Hin*d III (*Hsu* I), respectively. Identical conditions are employed except that selection of recombinant clones is accomplished as described in Example 1. Recombinant clones having the Figure 3 sequence are obtained.

(H) Example 4(G) is repeated in which plasmids pBR315 and pBR316 are used in place of pBR322. The same conditions as described in Example 4(G) are utilized and identical results are obtained.

(I) Example 1, 3(A), 3(B) and 4(A)—(H) are repeated using *E. coli* RRI or *E. coli* HB101 in place of *E. coli* χ1776. All conditions are as described and identical results are obtained.

Example 5

For this example, the cDNA is prepared as described in Example 1. *Eco* RI restriction site linkers are blunt-end ligated to the cDNA as described by Valenzuela, P., et al., *supra*, and cleaved with *Eco* RI as described by Ullrich, A., et al., *supra*.

(A) Charon 16A DNA prepared as described by Blattner et al., *supra*, is utilized as the transfer vector. The cohesive ends are annealed by incubation for 60 minutes at 42°C in 0.1 M Tris-HCl, pH 8.0 and 10 mm MgCl$_2$. The vector is cleaved at the *Eco* RI restriction site with *Eco* RI endonuclease and then treated with alkaline phosphatase as described by Ullrich, A., et al., *supra*. After ethanol precipitation, the phosphatase-treated vector DNA is added to cDNA containing *Eco* RI cohesive termini at a molar ratio of 2 moles vector to 1 mole cDNA. The mixture is ligated with T4 DNA ligase as described by Ullrich, A., et al., *supra*. The ligation mixture is added directly to a suspension of *E. coli* χ1776 cells prepared for transformation as described in Example 1 and the transformation is performed as described in Example 1. Recombinant phages are recovered and plated on Lac⁻ bacteria on plates containing 5-chloro-4-bromo-3-indolyl-$\beta$-D-glactoside (X6), (80 $\mu$g/ml) recombinant phages containing the cDNA inserted into the *Eco* RI site of Charon 16A are selected for the production of colorless plaques. The desired recombinants are isolated as described in Example 1 and digested with an excess of *Eco* RI endonuclease and the digest analyzed as described in Example 2. DNA's of about 700 base pairs in length and having the sequences shown in Figure 3 are recovered. Alternatively, the ligation mixture is utilized to form recombinant phages by *in vitro* packaging as described by Sternberg, N. et al., *Gene 1,* 255 (1977). Recombinant phages can also be screened by utilizing the *in situ* plaque hybridization technique of Benton, W. D. and Davis, R. W., *Science 196,* 180 (1977).

(B) Charon 3A DNA or Charon 4A DNA, prepared as described by Blattner, et al., *supra*, is used as the transfer vector in place of the Charon 16A DNA used above. All conditions are identical to those described for Charon 16A DNA. Selection of recombinant phages is performed by (a) plating the phages on Lac⁺ bacteria on plates containing X6 and isolating colorless plaques followed by either hybridization to a suitable probe or restriction endonuclease digestion as described by Blattner, et al., *supra*. The inserts are removed as described above, yielding DNA's of about 700 base pairs in length and having the sequences shown in Figure 3.

(C) $\lambda$gtWES · $\lambda$B DNA, prepared as described by Tiemier et al., *supra*, is utilized as the transfer vector in place of the Charon 16A used above. All conditions are identical to those described for Charon 16A. Selection of recombinant phages is performed by the hybridization method of Benton and Davis, *supra*. The inserts are removed as described above, yielding DNA's of about 700 base pairs in length and having the sequences shown in Figure 3.

(D) Examples 5(A)—(C) are repeated in which *E. coli* RPI, *E. coli* HB101, *E. coli* DP50 or *E. coli* DP50SupF is used in place of *E. coli* χ1776. All conditions are identical and identical results are obtained.

Example 6

For this example, the cDNA is prepared as described in Example 1. *Hin*d III restriction site linkers are added and the cDNA digested with *Hin*d III or *Hsu* I as described in Example 4(A).

The plasmid pC194 isolated from *S. aureus* as described by Ehrlich, S. D., *supra*, is utilized as the transfer vector. pC194 is cleaved at the *Hin*d III site with *Hin*d III or *Hsu* I and then treated with alkaline phosphatase as described by Ullrich, A. et al., *supra*. The cDNA having *Hin*d III cohesive termini is ligated to the cleaved pC194 as described by Ullrich, A., et al., *supra*. Competence-induction of *B. subtilis*

RUB331 is performed as described by Sgaramella, V., et al., *J. Mol. Biol., 105,* 587 (1976) and Borenstein, S. and Ephrati-Elizue, E., *J. Mol. Biol., 45,* 137 (1969). Transformation of *B. subtilis* RUB 331 is performed using the ligation mixture as described by Sgaramella et al. and Borenstein et al. The cell suspension is plated directly on L plates containing 3 μg of chloramphenicol. Selected recombinants are isolated as described in Example 1 and digested with *Hind* III and the digest analyzed as described in Example 2. DNA's of about 700 base pairs in length and having the sequences shown in Figure 3 are recovered.

Example 7
Synthesis of DNA coding for Prepro S1
(A) The insert DNA is first separated from pBR322/pLaS1 by *Pst* I endonuclease digestion, and purified by preparative gel electrophoresis. A 15 μg sample of purified insert DNA is then modified by suspending the DNA in water to which is added a concentrated solution of salts such that the final composition contains 70 mM tris pH 8.8, 70 mM MgCl$_2$ 10 mM dithiothreitol and 13.75 units of T4 polymerase (P—L Biochemicals, Milwaukee, Wisconsin) in a total volume of 250 μl. The reaction mixture is incubated 3.3 minutes at 37°C. To the reaction mixture is then added dTTP, 50 mM, to terminate endonucleolytic digestion at the next adenine residue in the sequence. After an additional 0.5 minutes' incubation, the reaction mixture is transferred to an ice-bath and the enzyme is inactivated by heat treatment for five minutes at 65°C. The treated DNA is recovered by ethanol precipitation. Digestion with S1 nuclease to provide blunt ends is carried out as described by Ullrich, A., et al., *Science, 196,* 1313 (1977).

The translated region of pLaS1 begins with an A residue, as shown in Figure 3. The nearest A residue in the 5'-untranslated region is five residues prior to the start codon (AUG). The above-described digestion conditions are designed to remove approximately 30 nucleotides from the 3' end. Therefore, a significant proportion of the T4 polymerase-digested DNA molecules will be terminated at the start codon at the position numbered −107.

(B) Example 7(A) is repeated in which the insert for Prepro S1 is removed by digestion of the transfer vectors prepared in Examples 3, 4, 5 and 6 by the appropriate restriction enzyme prior to digestion with T4 polymerase. The transfer vectors and restriction enzymes utilized are shown in Table 3.

TABLE 3

| Restriction enzyme | Transfer vector (Example) |
|---|---|
| *Pst* I | 3A, 3B, 4G, 4H |
| *Hind* III | 4A, 4B, 6 |
| *Sal* I | 4C, 4D |
| *Bam* HI | 4C, 4D |
| *Eco* RI | 4E, 4F, 5A, 5B, 5C |

The remaining steps are as described in Example 7(A) and identical results are obtained in each instance.

Example 8
Synthesis of DNA coding for Prepro S2
The plasmid pBR322/pLaS2 or the other transfer vectors containing the Prepro S2 insert prepared in the preceding examples is digested with the appropriate restriction enzyme as indicated in Example 7(A) or 7(B) and purified by preparative gel electrophoresis. Removal of the 5'-untranslated region is performed using T4 polymerase as described in Example 7(A) except that it is conducted in the presence of dATP. The reaction is stopped as described in Example 7(A). The DNA is extracted with phenol and precipitated. Excess dATP and the digested bases are removed by chromatography on 6—50 Sepadex. T4 polymerase is added to the DNA and incubated for 0.25 minutes before dATP is added to stop the digestion at the T preceding the start codon. The reaction is stopped and the DNA is extracted and chromatographed as described above. A final digestion with T4 polymerase in the presence of dTTP is conducted. The treated DNA is recovered by ethanol precipitation and digested with S1 nuclease to provide blunt ends as previously described. This yields a DNA which begins with the start codon at the position numbered −111.

Example 9
Synthesis of DNA coding for S1
The insert coding for Prepro S1 is removed by digestion of the transfer vectors prepared in the preceding Examples with the appropriate restriction enzymes as indicated in Example 7(A) or 7(B). The insert is then digested with *Xma* I and purified by preparative gel electrophoresis. The purified insert DNA is then digested with T4 polymerase in the presence of dATP as described in Example 7(A). The reaction is stopped, the treated DNA recovered and digested with S1 nuclease to provide blunt ends as described in Example 7(A). This procedure yields a DNA coding for all of S1 except the two N-terminal amino acids. A nucleotide sequence coding for these two amino acids is chemically synthesized by the procedure of Itakura, K. et al., *J. Biol. Chem. 250,* 4592 (1975) and Itakura, K. et al., *J. Am. Chem. Soc. 97,* 7326 (1975). This sequence is then blunt-end ligated to the remainder of the S1 coding sequence by the procedure of Valenzuela, P., et al., *Nature 280,* 815 (1979). This yields the complete coding sequence for S1.

Example 10
Synthesis of DNA coding for S2
The insert coding for Prepro S2 is removed by digestion of the transfer vectors prepared in the preceding Examples with the appropriate restriction enzymes as indicated in Example 7(A) or 7(B). The insert is then digested with the restriction enzyme *Ava* II or *Hgi* CII or *Hgi* EII. A fragment of DNA of about 120 base pairs in length which begins at the third base in the codon at position −15 of Prepro S2 and continues into the 3′ untranslated region is isolated by gel electrophoresis. This fragment is then digested with T4 polymerase as described in Examples 7(A) and 9 using five cycles following the format described in Example 8. These cycles are conducted in the presence of dTTP, dCTP, dGTP, dTTP and dCTP, respectively. After the final T4 polymerase digestion, the DNA is recovered by ethanol precipitation and digested with S1 nuclease to provide blunt ends as described in Example 7(A). This yields the complete coding sequence for S2.

Example 11
(A) The deoxynucleotide sequence coding for Prepro S1 prepared in Examples 7(A) and 7(B), i.e., Prepro S1 beginning at the start codon, is inserted into transfer vectors by following procedures described in Examples 1, 3(A), 3(B), 4(A)—(I), 5(A), 5(B) and 6. All conditions are identical to those described. The DNA is removed with the appropriate restriction enzyme, for example as shown in Table 3, and analyzed as described in Example 2. A DNA having the sequence beginning at the start codon at position −107 and continuing to the end of the sequence shown for pLaS1 in Figure 3 is obtained in each instance.
(B) The deoxynucleotide sequence coding for Prepro S2 prepared in Example 8, i.e., Prepro S2 beginning at the start codon, is inserted into transfer vectors, removed and analyzed as described in Example 11(A). A DNA having the sequence beginning at the start codon at position −111 and continuing to the end of the sequence shown for pLaS2 in Figure 3 is obtained in each instance.
(C) The deoxynucleotide sequence coding for S1 prepared in Example 9 is inserted into transfer vectors, removed and analyzed as described in Example 11(A). A DNA having the sequence shown for S1 (Somatostatin I) in Figure 3 is obtained in each instance.
(D) The deoxynucleotide sequence coding for S2 prepared in Example 10 is inserted into transfer vectors, removed and analyzed as described in Example 11(A). A DNA having the sequence shown for S2 (Somatostatin II) in Figure 3 is obtained in each instance.

Example 12
The various coding regions described herein can be expressed by any of the methods described above.
(A) Expression of Prepro S1 as a fusion protein is demonstrated by incorporation of a radioactive amino acid into a protein not present in untransformed cells or by radioimmunoassay. In both instances, bacterial cells containing pBR322/pLaS1, prepared in Example 1, or bacterial cells containing pBR322 as the control are grown in nutrient broth. In the former procedure, after three hours of growth, 1.5 ml of cells are radioactively labelled by addition of 20 $\mu$ Ci $^{35}$S—L methionine and incubated for 10 minutes. The cells are then collected by centrifugation, washed and resuspended in 250 $\mu l$ of buffer containing glycol 10% (v/v), $\beta$-mercaptoethanol 5% (v/v), and SDS 2.3% (w/v) in 0.0625 M tris pH 6.8. The suspension is boiled for five minutes, then applied to a 10% (w/v) SDS-polyacrylamide gel and fractionated by electrophoresis. The protein bands are visualized by autoradiography. The results show the existence of a new protein band not observed in non-transformed cultures.
In the radioimmunoassay procedure, the bacterial cells are collected after growth by centrifugation. The cells are resuspended, lysed and radiolabelled antibody to S1 that does not depend upon a free N-terminal group at position 1 of S1 (Arimura, A., et al., *Proc. Soc. Exp. Biol. Med. 148,* 784 (1975)) is added. The immune complex is precipitated and radioactivity measured. A position reaction is obtained, indicating the production of a fusion protein containing Prepro S1.
(B) For the direct expression of Prepro S1, the insert pLaS1 is modified as described in Example 7(A) or 7(B) or the modified insert is removed from the transfer vectors prepared in Example 11(A).
A vector for direct expression is constructed by modification of the plasmid p*trp*E30 by the removal of 23—29 nucleotides using T4 polymerase and S1 nuclease as described above.
Both the modified pLaS1 insert and the modified expression vector are provided with a specific linker oligonucleotide having the sequence

5′—CCGGATCCGG—3′

on one strand and its complementary sequence on the other, by blunt end ligation using DNA ligase as described by Valenzuela et al., *Nature, 280,* 815 (1979). The linker oligonucleotides provide restriction sites sensitive to *Bam* HI endonuclease which are employed to facilitate insertion. The insertion reaction is carried out essentially as described by Ullrich, A., et al., *supra.* Host bacteria, *E. coli* HB101, RRI or χ1776, are transformed by the recombinant vectors bearing the inserted modified Prepro S1 coding region, and transformants are selected for resistance to ampicillin. A single transformant selected for further analysis is designated ptrpE30/PPS1.

Bacterial cells transformed by ptrpE30/PPS1 are grown in a standard minimal medium (M9) supplemented with leucine, proline, vitamin B1 and ampicillin, at 37°C. In early log phase, the *trp* operon is induced by addition of $\beta$-indolyl-acrylic acid (30 $\mu$g/ml of medium). Control cultures are left uninduced. After three more hours of growth, 1.5 ml of cells are radioactively labeled by addition of 20 $\mu$ Ci $^{35}$S-L-methionine and incubation for 10 minutes. The cells are then collected by centrifugation, washed and resuspended in 250 $\mu$l of buffer containing blycol 10% (v/v), $\beta$-mercapto-ethanol 5% (v/v), and SDS 2.3% (w/v) in 0.0625M tris pH 6.8. The suspension is boiled for five minutes, then applied to a 10% (w/v) SDS-polyacrylamide gel and fractionated by electrophoresis. The protein bands are visualized by autoradiography. The results show the existence of a new protein band of about 13,000 molecular weight, not observed in uninduced or non-transformed cultures.

Prepro S1 is purified by conventional techniques including, for example, gel filtration, ion exchange chromatography, affinity chromatography, and differential solubility techniques. Prepro S1 is converted to Pro S1 by following the procedure described by Jackson, R. C., et al., *supra.*

(C) Prepro S2 is expressed either as a fusion protein or directly by following the procedure described in Example 12(A) or 12(B), respectively, using pBR322/pLaS2 or the modified pLaS2 insert, respectively. Production of a fusion protein containing Prepro S2 or production of Prepro S2 directly is determined by radioactive amino acid incorporation. The procedure is identical to that described in Example 12(A) and 12(B) except that radiolabelled tyrosine is used in place of the radiolabelled methionine. The results show the existence of a protein band not observed in non-transformed cultures for the production of a fusion protein, and the existence of a protein band of about 14,100 molecular weight not observed in uninduced or non-transformed cultures for the production of Prepro S2.

(D) A specific linker having the sequence

5'—CCGGATCCGGATG—3'

on one strand and its complementary sequence on the other is blunt-end ligated to the DNA coding for S1 or S2 as prepared in Examples 9 and 10, respectively. This modified DNA is then inserted in the modified plasmid ptrpE30 as described in Example 12(B). Host bacteria are transformed and cultured and analyzed as described in Example 12(B) or 12(C), respectively. The results indicate the production of S1 and S2. This is confirmed by an amino acid analysis.

While the invention has been described in connection with specific embodiments thereof, it will be understood that it is capable of further modifications. This application is intended to cover any variations, uses or adaptations of the invention following, in general, the principles of the invention and including such departures from the present disclosure as come within known and customary practice within the art to which the invention pertains.

## Claims for the Contracting States BE CH DE FR GB IT LI LU NL SE

1. A somatostatin or somatostatin precursor selected from the group comprising prepro-somatostatin-1, prosomatostatin-1, pre-prosomatostatin-2, prosomatostatin-2 and somatostatin-2.

2. A DNA transfer vector comprising a deoxynucleotide sequence coding for a somatostatin or somatostatin precursor selected from the group comprising preprosomatostatin-1, prosomatostatin-1, preprosomatostatin-2, prosomatostatin-2 and somatostatin-2.

3. The DNA transfer vector of claim 2 wherein the somatostatin is somatostatin-2.

4. The DNA transfer vector of claim 3 wherein said deoxynucleotide sequence comprises a plus strand having the sequence:

5'—GCT GGC TGT AAG AAC TTC TAT TGG AAG GGC TTC ACT TCC TGT TAA—3'

wherein
    A is deoxyadenyl,
    G is deoxyguanyl,
    C is deoxycytosyl and
    T is thymidyl.

5. The DNA transfer vector of claim 2 wherein the somatostatin precursor is preprosomatostatin-1.

6. The DNA transfer vector of claim 5 wherein said deoxynucleotide sequence comprises a plus strand having the sequence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC GCC CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

wherein
  A is deoxyadenyl,
  G is deoxyguanyl,
  C is deoxycytosyl and
  T is thymidyl.

7. The DNA transfer vector of claim 2 wherein the somatostatin precursor is preprosomatostatin-2.

8. The DNA transfer vector of claim 7 wherein said deoxynucleotide sequence comprises a plus strand having the sequence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

wherein
  A is deoxyadenyl,
  G is deoxyguanyl,
  C is deoxycytosyl and
  T is thymidyl.

9. The DNA transfer vector of any of claims 2 to 8 wherein said deoxynucleotide sequence comprises a DNA strand having a deoxynucleotide sequence complementary to the ribonucleotide sequence of mRNA coding for said somatostatin or somatostatin precursor.

10. The transfer vector of claim 5 or 7 comprising pBR322/pLaS1 or pBR322/pLaS2.

11. A microorganism transformed by the transfer vector of any of claims 2 to 10.

12. The microorganism of claim 11 selected from the group comprising *Escherichia coli* HB101, RR1 or $\chi$1776.

13. A process for preparing a DNA transfer vector for use in maintaining and replicating a deoxynucleotide sequence, said process comprising reacting said deoxynucleotide sequence with a DNA molecule prepared by cleaving a transfer vector with a restriction enzyme, characterized in that the deoxynucleotide sequence codes for a somatostatin or a somatostatin precursor selected from the group comprising preprosomatostatin-1, prosomatostatin-1, preprosomatostatin-2, prosomatostatin-2 and somatostatin-2.

14. The process of claim 13 wherein said deoxynucleotide sequence codes for somatostatin-2.

15. The process of claim 14 wherein said deoxynucleotide sequence coding for somatostatin-2 comprises a plus strand having the sequence:

5'—GCT GGC TGT AAG AAC TTC TAT TGG AAG GGC TTC ACT TCC TGT TAA—3'

wherein
  A is deoxyadenyl,
  G is deoxyguanyl,
  C is deoxycytosyl and
  T is thymidyl.

16. The process of claim 13 wherein said deoxynucleotide sequence codes for preprosomatostatin-1.

17. The process of claim 16 wherein said deoxynucleotide sequence coding for preprosomatostatin-1 comprises a plus strand having the sequence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC CGG CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

wherein
  A is deoxyadenyl,
  G is deoxyguanyl,
  C is deoxycytosyl and
  T is thymidyl.

18. The process of claim 13 wherein said deoxynucleotide sequence codes for preprosomatostatin-2.

19. The process of claim 18 wherein said deoxynucleotide sequence coding for preprosomatostatin-2 comprises a plus strand having the sequence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

wherein
   A is deoxyadenyl,
   G is deoxyguanyl,
   C is deoxycytosyl and
   T is thymidyl.

20. A process for making the plasmid pBR322/pLaS1 or pBR322/pLaS2 according to claim 13 characterized in that the DNA molecule prepared by cleaving a transfer vector with a restriction enzyme is pBR322 and the restriction enzyme is *Pst* I.

21. A process for making an expression transfer vector in accordance with claim 13 for use in expressing a deoxynucleotide sequence coding for a somatostatin or somatostatin precursor characterized in that the DNA molecule prepared by cleaving a transfer vector with a restriction enzyme is cleaved at a point within an expressible control region.

22. A process for making a fusion protein, comprising the amino acid sequence of a somatostatin or a somatostatin precursor as its C-terminal end and a portion of a procaryotic protein as its N-terminal end, said process comprising incubating a microorganism transformed by an expression vector prepared in accordance with claim 21.

23. A process for synthesizing a somatostatin or a somatostatin precursor selected from the group comprising preprosomatostatin-1, preprosomatostatin-2 and somatostatin-2, said process comprising incubating a microorganism transformed by an expression vector comprising a deoxynucleotide sequence coding for said somatostatin or somatostatin precursor prepared in accordance with any of claims 14 to 19 under conditions suitable for expression of said deoxynucleotide sequence and purifying said somatostatin or somatostatin precursor from the lystate or culture medium of said microorganism.

**Claims for the Contracting State AT**

1. A process for preparing a DNA transfer vector for use in maintaining and replicating a deoxynucleotide sequence, said process comprising reacting said deoxynucleotide sequence with a DNA molecule prepared by cleaving a transfer vector with a restriction enzyme, characterized in that the deoxynucleotide sequence codes for a somatostatin or a somatostatin precursor selected from the group comprising preprosomatostatin-1, prosomatostatin-1, preprosomatostatin-2, prosomatostatin-2 and somatostatin-2.

2. The process of claim 1 wherein said deoxynucleotide sequence codes for somatostatin-2.

3. The process of claim 2 wherein said deoxynucleotide sequence coding for somatostatin-2 comprises a plus strand having the sequence:

5'—GCT GGC TGT AAG AAC TTC TAT TGG AAG GGC TTC ACT TCC TGT TAA—3'

wherein
   A is deoxyadenyl,
   G is deoxyguanyl,
   C is deoxycytosyl and
   T is thymidyl.

4. The process of claim 1 wherein said deoxynucleotide sequence codes for preprosomatostatin-1.

5. The process of claim 4 wherein said deoxynucleotide sequence coding for preprosomatostatin-1 comprises a plus strand having the sequence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC GCC CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

wherein
   A is deoxyadenyl,
   G is deoxyguanyl,
   C is deoxycytosyl and
   T is thymidyl.

6. The process of claim 1 wherein said deoxynucleotide sequence codes for preprosomatostatin-2.

7. The process of claim 6 wherein said deoxynucleotide sequence coding for preprosomatostatin-2 comprises a plus strand having the sequence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

wherein
   A is deoxyadenyl,

G is deoxyguanyl,

C is deoxycytosyl and

T is thymidyl.

8. A process for making the plasmid pBR322/pLaS1 or pBR322/pLaS2 according to claim 1 characterized in that the DNA molecule prepared by cleaving a transfer vector with a restriction enzyme is pBR322 and the restriction enzyme is *Pst* I.

9. A process for making an expression transfer vector in accordance with claim 1 for use in expressing a deoxynucleotide sequence coding for a somatostatin or somatostatin precursor characterized in that the DNA molecule prepared by cleaving a transfer vector with a restriction enzyme is cleaved at a point within an expressible control region.

10. A process for making a fusion protein comprising the amino acid sequence of a somatostatin or a somatostatin precursor as its C-terminal end and a portion of a procaryotic protein as its N-terminal end, said process comprising incubating a microorganism transformed by an expression vector prepared in accordance with claim 9.

11. A process for synthesizing a somatostatin or a somatostatin precursor selected from the group comprising preprosomatostatin-1, preprosomatostatin-2 and somatostatin-2, said process comprising incubating a microorganism transformed by an expression vector comprising a deoxynucleotide sequence coding for said somatostatin or somatostatin precursor prepared in accordance with any of claims 2 to 7 under conditions suitable for expression of said deoxynucleotide sequence and purifying said somatostatin or somatostatin precursor from the lysate or culture medium of said microorganism.

**Patentansprüche für die Vertragstaaten: BE, CH, DE, FR, GB, IT, LI, LU, NL, SE**

1. Somatostatin oder Vorgänger des Somatostatins, ausgewählt aus der Gruppe umfassend Preprosomatostatin-1, Prosomatostatin-1, Preprosomatostatin-2, Prosomatostatin-2 und Somatostatin-2.

2. Ein DNA Übertragungsvektor, enthaltend eine Deoxynukleotidsequenz, welche für Somatostatin oder einen Vorgänger des Somatostatins, ausgewählt aus der Gruppe umfassend Preprosomatostatin-1, Prosomatostatin-1, Preprosomatostatin-2, Prosomatostatin-2 und Somatostatin-2, kodiert.

3. Der DNA Übertragungsvektor nach Anspruch 2, worin das Somatostatin Somatostatin-2 ist.

4. Der DNA Übertragungsvektor nach Anspruch 3, worin die Deoxynukleotidsequenz einen plus Strang mit der Sequenz:

5′—GCT GGC TGT AAG AAC TTC TAT TGG AAG GGC TTC ACT TCC TGT TAA—3′

enthält, worin

A Deoxyadenyl,

G Deoxyguanyl,

C Deoxycytosyl und

T Thymidyl bedeutet.

5. Der DNA Übertragungsvektor nach Anspruch 2, worin der Somatostatin-Vorgänger Preprosomatostatin-1 ist.

6. Der DNA Übertragungsvektor nach Anspruch 5, worin die Deoxynukleotidsequenz einen plus Strang mit der Sequenz:

5′—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC GCC CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3′

enthält, worin

A Deoxyadenyl,

G Deoxyguanyl,

C Deoxycytosyl und

T Thymidyl bedeutet.

7. Der DNA Übertragungsvektor nach Anspruch 2, worin der Somatostatin Vorgänger Preprosomatostatin-2 ist.

8. Der DNA Übertragungsvektor nach Anspruch 7, worin die Nukleotidsequenz einen plus Strang mit der Sequenz:

5′—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3′

enthält, worin

A Deoxyadenyl,

G Deoxaguanyl,

C Deoxycytosyl und

T Thymidyl bedeutet.

9. Der DNA Übertragungsvektor nach einem der Ansprüche 2—8, worin die Nukleotidsequenz einen DNA Strang aufweist, dessen Deoxynukleotid sequenz komplementär zur Ribonukleotid Sequenz von mRNA ist, welche für Somatostatin oder einen Vorgänger des Somatostatins kodiert.

10. Der Übertragungsvektor nach Anspruch 5 oder 7, enthaltend pBR322/pLaS1 oder pBR322/pLAS2.

11. Ein Mikroorganismus, der durch den Übertragungsvektor nach einem der Ansprüche 2—10 transformiert worden ist.

12. Der Mikroorganismus nach Anspruch 11, ausgewählt aus der Gruppe umfassend *Escherichia coli* HB101, RR1 oder $\chi$1776.

13. Verfahren zur Herstellung eines DNA Übertragungsvektors zur Verwendung bei der Aufrechterhaltung und Replizierung einer Deoxynukleotidsequenz, wobei dieses Verfahren die Umsetzung der Deoxynukleotidsequenz mit einem DNA Molekül umfaßt, hergestellt durch Spaltung eines Übertragungsvektors mit einem Restriktionsenzym, dadurch gekennzeichnet, daß die Deoxynukleotidsequenz für ein Somatostatin oder einen Vorgänger des Somatostatins kodiert, ausgewählt aus der Gruppe umfassend Preprosomatostatin-1, Prosomatostatin-1, Preprosomatostatin-2, Prosomatostatin-2 und Somatostatin-2.

14. Verfahren nach Anspruch 13, worin die Deoxynukleotidsequenz für Somatostatin-2 kodiert.

15. Verfahren nach Anspruch 14, worin die Deoxynukleotidsequenz die für Somatostatin-2 kodiert einen plus Strang mit der Sequenz:

5′—GCT GGC TGT AAG AAC TTC TAT TGG AAG GGC TTC ACT TCC TGT TAA—3′

enthält, worin
　A Deoxyadenyl,
　G Deoxyguanyl,
　C Deoxycytosyl und
　T Thymidyl bedeutet.

16. Verfahren nach Anspruch 13, wobei die Deoxynukleotidsequenz für Preprosomatostatin-1 kodiert.

17. Verfahren nach Anspruch 16, worin die Deoxynukleotidsequenz, die für Preprosomatostatin-1 kodiert, einen plus Strang mit der Sequenz:

5′—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC CGG CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3′

enthält, worin

A Deoxyadenyl,
G Deoxyguanyl,
C Deocycytosyl und
T Thymidyl bedeutet.

18. Verfahren nach Anspruch 13, worin die Deoxynukleotidsequenz für Preprosomatostatin-2 kodiert.

19. Verfahren nach Anspruch 18, worin die Deoxynukleotidsequenz, die für Preprosomatostatin-2 kodiert, einen plus Strang mit der Sequenz:

5′—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3′

enthält, worin
　A Deoxyadenyl,
　G Deoxyguanyl,
　C Deoxycytosyl und
　T Thymidyl bedeutet.

20. Verfahren zur Herstellung des Plasmids pBR322/pLaS1 oder pBR322/pLaS2 nach Anspruch 13, dadurch gekennzeichnet, daß das DNA Molekül, hergestellt durch Spaltung eines Übertragungsvektors mti einem Restriktionsenzym, pBR322 und das Restriktionsenzym *Pst* I ist.

21. Verfahren zur Herstellung eines Expressions-Übertragungsvektors nach Anspruch 13 zur Verwendung bei der Expression einer Nukleotid Sequenz, kodierend für ein Somatostatin oder einen Vorgänger des Somatostatins, dadurch gekennzeichnet, daß das DNA Molekül, hergestellt durch Spaltung eines Übertragungsvektors mit einem Restriktionsenzym, an einem Punkt innerhalb eines expressionsfähigen Kontrollbereichs gespalten wird.

22. Verfahren zur Herstellung eines Fusionsproteins, enthaltend die Aminosäuresequenz eines Somatostatins oder eines Vorgängers des Somatostatins an seinem C-terminalen Ende und einen Teil eines procaryotischen Proteins an seinem N-terminalen Ende, wobei dieses Verfahren die Züchtung eines Mikroorganismus umfaßt, der mittels eines Expressionsvektors, hergestellt nach Anspruch 21, transformiert worden ist.

23. Verfahren zur Synthetisierung eines Somatostatins oder Vorgänger des Somatostatins, ausgewählt aus der Gruppe umfassend Preprosomatostatin-1, Preprosomatostatin-2 und Somatostatin-2, wobei dieses Verfahren die Züchtung eines Mikroorganismus umfaßt, der mittels eines Expressionsvektors, enthaltend eine Deoxynukleotidsequenz, kodierend für dieses Somatostatin oder den Vorgänger des Somatostatins, hergestellt nach einem der Ansprüche 14—19, transformiert worden ist, und zwar unter geeigneten Bedingungen zur Expression der genannten Deoxynukleotidsequenz und Reinigung des Somatostatins oder des Vorgängers des Somatostatins vom Lysat oder Züchtungsmedium des genannten Mikroorganismus.

**Patentansprüche für den Vertragsstraat AT**

1. Verfahren zur Herstellung eines DNA Übertragungsvektors zur Verwendung bei der Aufrechterhaltung und Replizierung einer Deoxynukleotidsequenz, wobei dieses Verfahren die Umsetzung der Deoxynukleotidsequenz mit einem DNA Molekül umfaßt, hergestellt durch Spaltung eines Übertragungsvektors mit einem Restriktionsenzym, dadurch gekennzeichnet, daß die Deoxynukleotidsequenz für ein Somatostatin oder einen Vorgänger des Somatostatins kodiert, ausgewählt aus der Gruppe umfassend Preprosomatostatin-1, Prosomatostatin-1, Preprosomatostatin-2, Prosomatostatin-2 und Somatostatin-2.

2. Verfahren nach Anspruch 1, worin die Deoxynukleotidsequenz für Somatostatin-2 kodiert.

3. Verfahren nach Anspruch 2, worin die Deoxynukleotidsequenz die für Somatostatin-2 kodiert einen plus Strang mit der Sequenz:

5′—GCT GGC TGT AAG AAC TTC TAT TGG AAG GGC TTC ACT TCC TGT TAA—3′

enthält, worin
A Deoxyadenyl,
G Deoxyguanyl,
C Deoxycytosyl und
T Thymidyl bedeutet.

4. Verfahren nach Anspruch 1, wobei die Deoxynukleotidsequenz für Preprosomatostatin-1 kodiert.

5. Verfahren nach Anspruch 4, worin die Deoxynukleotidsequenz, die für Preprosomatostatin-1 kodiert, einen plus Strang mit der Sequenz:

5′—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC CGG CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3′

enthält, worin
A Deoxyadenyl,
G Deoxyguanyl,
C Deocycytosyl und
T Thymidyl bedeutet.

6. Verfahren nach Anspruch 1, worin die Deoxynukleotidsequenz für Preprosomatostatin-2 kodiert.

7. Verfahren nach Anspruch 6, worin die Deoxynukleotidsequenz, die für Preprosomatostatin-2 kodiert, einen plus Strang mit der Sequenz:

5′—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3′

enthält, worin
A Deoxyadenyl,
G Deoxyguanyl,
C Deoxycytosyl und
T Thymidyl bedeutet.

8. Verfahren zur Herstellung des Plasmids pBR322/pLaS1 oder pBR322/pLaS2 nach Anspruch 1, dadurch gekennzeichnet, daß das DNA Molekül, hergestellt durch Spaltung eines Übertragungsvektors mit einem Restriktionsenzym, pBR322 und das Restriktionsenzym *Pst* I ist.

9. Verfahren zur Herstellung eines Expressions-Übertragungsvektors nach Anspruch 1 zur Verwendung bei der Expression einer Nukleotid Sequenz, kodierend für ein Somatostatin oder einen Vorgänger des Somatostatins, dadurch gekennzeichnet, daß das DNA Molekül, hergestellt durch Spaltung eines Übertragungsvektors mit einem Restriktionsenzym, an einem Punkt innerhalb eines expressionsfähigen Kontrollbereichs gespalten wird.

10. Verfahren zur Herstellung eines Fusionsproteins, enthaltend die Aminosäuresequenz eines Somatostatins oder eines Vorgängers des Somatostatins an seinem C-terminalen Ende und einen Teil eines procaryotischen Proteins an seinem N-terminalen Ende, wobei diese Verfahren die Züchtung eines Mikroorganismus umfaßt, der mittels eines Expressionsvektors, hergestellt nach Anspruch 9, transformiert worden ist.

11. Verfahren zur Synthetisierung eines Somatostatins oder Vorgänger des Somatostatins, ausgewählt aus der Gruppe umfassend Preprosomatostatin-1, Preprosomatostatin-2 und Somatostatin-2, wobei diese Verfahren die Züchtung eines Mikroorganismus umfaßt, der mittels eines Expressionsvektors, enthaltend eine Deoxynukleotidsequenz, kodierend für dieses Somatostatin oder den Vorgänger des Somatostatins, hergestellt nach einem der Ansprüche 2—7, transformiert worden ist, und zwar unter geeigneten Bedingungen zur Expression der genannten Deoxynukleotidsequenz und Reinigung des Somatostatins oder des Vorgängers des Somatostatins vom Lysat oder Züchtungsmedium des genannten Mikroorganismus.

**Revendications pour les Etats Contractants BE, CH, DE, FR, GB, IT, LI, LU, NL, SE**

1. Somatostatine ou précurseur de somatostatine choisi parmi la préprosomatostatine-1, la prosomatostatine-1, la préprosomatostatine-2, la prosomatostatine-2 et la somatostatine-2.

2. Vecteur de transfer d'ADN comprenant une séquence de désoxynucléotides codant pour une somatostatine ou un précurseur de somatostatine choisi parmi la préprosomatostatine-1, la prosomatostatine-1, la préprosomatostatine-2, la prosomatostatine-2 et la somatostatine-2.

3. Vecteur de transfert d'ADN selon la revendication 2, caractérisé en ce que la somatostatine est la somatostatine-2.

4. Vecteur de transfert d'ADN selon la revendication 3, caractérisé en ce que la séquence de désoxynucléotides comprend un cordon plus ayant la séquence:

5'—GCT GGC TGT AAG AAC TTC TAT TGG AAG GGC TTC ACT TCC TGT TAA—3'

dans laquelle
    A est désoxyadényle,
    G est désoxyguanyle,
    C est désoxycytosyle et
    T est thymidyle.

5. Vecteur de transfert d'ADN selon la revendication 2, caractérisé en ce que le précurseur de somatostatine est la préprosomatostatine-1.

6. Vecteur de transfert d'ADN selon la revendication 5, caractérisé en ce que la séquence de désoxynucléotides comprend un cordon plus ayant la séquence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC GCC CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

dans laquelle
    A est désoxyadényle,
    G est désoxyguanyle,
    C est désoxycytosyle et
    T est thymidyle.

7. Vecteur de transfert selon la revendication 2, dans lequel le précurseur de somatostatine est la préprosomatostatine-2.

8. Vecteur de transfert selon la revendication 7, caractérisé en ce que la séquence de désoxynucléotides comprend un cordon plus ayant la séquence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

dans laquelle
    A est désoxyadényle,
    G est désoxyguanyle,
    C est désoxycytosyle et
    T est thymidyle.

9. Vecteur de transfert d'ADN selon l'une quelconque des revendications 2 à 8, caractérisé en ce que la séquence de désoxynucléotides comprend un cordon d'ADN ayant une séquence de désoxynucléotides complémentaire de la séquence de ribonucléotides d'ARNm codant pour la somatostatine ou le précurseur de somatostatine.

10. Vecteur de transfert selon la revendication 5 ou 7, comprenant pBR322/pLaS1 et pBR322/pLaS2.

11. Microorganisme transformé par le vecteur de transfert selon l'une quelconque des revendications 2 à 10.

12. Microorganisme selon la revendication 11, choisi parmi Escherichia coli HB 101, RR1 ou $\chi$1776.

13. Procédé de préparation d'un vecteur de transfert d'ADN utilisable pour la conservation et la réplique d'une séquence de désoxynucléotides, dans lequel on fait réagir la séquence de désoxynucléotides avec une molécule d'ADN préparée par scission d'un vecteur de transfert avec une enzyme de restriction, caractérisé en ce que la séquence de désoxynucléotides code pour une somatostatine ou un précurseur de somatostatine choisi parmi la préprosomatostatine-1, la prosomatostatine-1, la préprosomatostatine-2, la prosomatostatine-2 et la somatostatine-2.

14. Procédé selon la revendication 13, caractérisé en ce que la séquence de désoxynucléotides code pour la somatostatine-2.

15. Procédé selon la revendication 14, caractérisé en ce que la séquence de désoxynucléotides codant pour la somatostatine-2 comprend un cordon plus ayant la séquence:

5'—GCT GGC TGT AAG AAC TTC TAT TGG AAG GGC TTC ACT TCC TGT TAA—3'

dans laquelle
    A est désoxyadényle,
    G est désoxyguanyle,
    C est désoxycytosyle et
    T est thymidyle.

16. Procédé selon la revendication 13, caractérisé en ce que la séquence de désoxynucléotides code pour la préprosomatostatine-1.

17. Procédé selon la revendication 16, caractérisé en ce que la séquence de désoxynucléotides codant pour la préprosomatostatine-1 comprend un cordon plus ayant la séquence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC CGG CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

dans laquelle
    A est désoxyadényle,
    G est désoxyguanyle,
    C est désoxycytosyle et
    T est thymidyle.

18. Procédé selon la revendication 13, caractérisé en ce que la séquence de désoxynucléotides code pour la préprosomatostatine-2.

19. Procédé selon la revendication 18, caractérisé en ce que la séquence de désoxynucléotides codant pour la préprosomatostatine-2 comprend un cordon plus ayant la séquence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

dans laquelle
    A est désoxyadényle,
    G est désoxyguanyle,
    C est désoxycytosyle et
    T est thymidyle.

20. Procédé de production du plasmide pBR 322/pLa S1 ou pBR 322/pLa S2 selon la revendication 13, caractérisé en ce que la molécule d'ADN préparée par scission d'un vecteur de transfert avec une enzyme de restriction est pBR 222 et l'enzyme de restriction est *Pst* I.

21. Procédé de production d'un vecteur de transfert d'expression selon la revendication 13, utilisable dans l'expression d'une séquence de désoxynucléotides codant pour une somatostatine ou un précurseur de somatostatine, caractérisé en ce que la molécule d'ADN préparée par scission d'un vecteur de transfert avec une enzyme de restriction est scindée en un point situé au sein d'une région de régulation exprimable.

22. Procédé de production d'une protéine de fusion comprenant la séquence d'acides aminés d'une somatostatine ou d'un précurseur de somatostatine en tant qu'extrémité C-terminale et une portion d'une protéine procaryote en tant qu'extrémité N-terminale, le procédé comprenant une incubation d'un microorganisme transformé par un vecteur d'expression préparé selon la revendication 21.

23. Procédé de synthese d'un somatostatine ou d'un précurseur de somatostatine choisi parmi la préprosomatostatine-1, la préprosomatostatine-2 et la somatostatine-2, le procédé comprenant une incubation d'un microorganisme transformé par un vecteur d'expression comprenant une séquence de désoxynucléotides codant pour la somatostatine ou le précurseur de somatostatine, préparée selon l'une quelconque des revendications 14 à 19, dans des conditions appropriées pour l'expression de la séquence de désoxynucléotides, et une purification de la somatostatine ou du précurseur de somatostatine à partir du lysat ou du milieu de culture du microorganisme.

**Revendications pour l'Etat Contractant: AT**

1. Procédé de préparation d'un vecteur de transfert d'ADN utilisable pour la conservation et la réplique d'une séquence de désoxynucléotides, dans lequel on fait réagir la séquence de désoxynucléotides avec une molécule d'ADN préparéee par scission d'un vecteur de transfert avec une enzyme de restriction, caractérisé en ce que la séquence de désoxynucléotides code pour une somatostatine ou un précurseur de somatostatine choisi parmi la préprosomatostatine-1, la prosomatostatine-1, la préprosomatostatine-2, la prosomatostatine-2 et la somatostatine-2.

2. Procédé selon la revendication 1, caractérisé en ce que la séquence de désoxynucléotides code pour la somatostatine-2.

3. Procédé selon la revendication 2, caractérisé en ce que la séquence de désoxynucléotides codant pour la somatostatine-2 comprend un cordon plus ayant la séquence:

5'—GCT GGC TGT AAG AAC TTC TAT TGG AAG GGC TTC ACT TCC TGT TAA—3'

dans laquelle
    A est désoxyadényle,
    G est désoxyguanyle,
    C est désoxycytosyle et
    T est thymidyle.

4. Procédé selon la revendication 1, caractérisé en ce que la séquence de désoxynucléotides code pour la préprosomatostatine-1.

5. Procédé selon la revendication 4, caractérisé en ce que la séquence de désoxynucléotides codant pour la préprosomatostatine-1 comprend un cordon plus ayant la séquence:

5'—ATG AAG ATG GTC TCC TCC TCG CGC CTC CGC TGC CTC CTC GTG CTC CTG CTG TCC CTG ACC GCC TCC ATC AGC TGC TCC TTC GCC GGA CAG AGA GAC TCC AAA CTC CGC CTG CTG CTG CAC CGG TAC CCG CTG CAG GGC TCC AAA CAG GAC ATG ACT CGC TCC GCC TTG GCC GAG CTG CTC CTG TCG GAC CTC CTG CAG GGG GAG AAC GAG GCT CTG GAG GAG GAG AAC TTC CCT CTG GCC GAA GGA GGA CCC GAG GAC GCC CAC GCC GAC CTA GAG CGG GCC GCC AGC GGG GGG CCT CTG CTC GCC CCC CGG GAG AGA AAG GCC GGC TGC AAG AAC TTC TTC TGG AAA ACC TTC ACC TCC TGC TGA—3'

dans laquelle
    A est désoxyadényle,
    G est désoxyguanyle,
    C est désoxycytosyle et
    T est thymidyle.

6. Procédé selon la revendication 1, caractérisé en ce que la séquence de désoxynucléotides code pour la préprosomatostatine-2.

7. Procédé selon la revendication 6, caractérisé en ce que la séquence de désoxynucléotides codant pour la préprosomatostatine-2 comprend un cordon plus ayant la séquence:

5'—ATG CAG TGT ATC CGT TGT CCC GCC ATC TTG GCT CTC CTG GCG TTG GTT CTG TGC GGC CCA AGT GTT TCC TCC CAG CTC GAC AGA GAG CAG AGC GAC AAC CAG GAC CTG GAC CTG GAG CTG CGT CAG CAC TGG CTG CTG GAG AGA GCC CGG AGC GCC GGA CTC CTG TCC CAG GAG TGG AGT AAA CGG GCG GTG GAG GAG CTG CTG GCT CAG ATG TCT CTG CCA GAG GCC ACG TTC CAG CGG GAG GCG GAG GAC GCG TCC ATG GCA ACA GAA GGA CGG ATG AAC CTA GAG CGG TCC GTG GAC TCT ACC AAC AAC CTA CCC CCT CGT GAG CGT AAA GCT GGC TGT AAG AAC TTC TAT TGG AAG GGC TTC ACT TCC TGT TAA—3'

dans laquelle
    A est désoxyadényle,
    G est désoxyguanyle,
    C est désoxycytosyle et
    T est thymidyle.

8. Procédé de production du plasmide pBR 322/pLa S1 ou pBR 322/PLa S2 selon la revendication 1, caractérisé en ce que la molécule d'ADN préparée par scission d'un vecteur de transfert avec une enzyme de restriction est pBR 322 et l'enzyme de restriction est *Pst* I.

9. Procédé de production d'un vecteur de transfert d'expression selon la revendication 1 utilisable dans l'expression d'une séquence de désoxynucléotides codant pour une somatostatine ou un précurseur de somatostatine, caractérisé en ce que la molécule d'ADN préparée par scission d'un vecteur de transfert avec une enzyme de restriction est scindé en un point situé au sein d'une région de régulation exprimable.

10. Procédé de production d'une protéine de fusion comprenant la séquence d'acides aminés d'une somatostatine ou d'un précurseur de somatostatine en tant qu'extrémité C-terminale et une portion d'une protéine procaryote en tant qu'extrémité N-terminale, le procédé comprenant une incubation d'un microorganisme transformé par un vecteur d'expression préparé selon la revendication 9.

11. Procédé de synthèse d'une somatostatine ou d'un précurseur de somatostatine choisi parmi la préprosomatostatine-1, la préprosomatostatine-2 et la somatostatine-2, le procédé comprenant une incubation d'un microorganisme transformé par un vecteur d'expression comprenant une séquence de désoxynucléotides codant pour la somatostatine ou le précurseur de somatostatine, préparée selon l'une quelconque des revendications 2 à 7 dans des conditions appropriées pour l'expression de la séquence de désoxynucléotides, et une purification de la somatostatine ou du précurseur de somatostatine à partir du lysat ou du milieu de culture du microorganisme.

# FIG. I

# FIG. 2

```
                                GAUCCGCAGACGCCGCCAGACGUACAGACAUCACGUG
                                •  •  •••••  •  •  ••••••   ••••••
CAGAGACAAACCCAGCAGAACCAGUAGAACCAGCAGAAGACACCAGACCAGCAGACAGU:::::

-107                          -100
met lys met val ser scr ser arg leu arg cys leu leu val leu leu
AUG AAG AUG GUC UCC UCC UCG CGC CUC CGC UGC CUC CUC GUG CUC CUG
•••  ••    ••    •    •    •   ••       ••• ••• • •   •    •
AUG CAG UGU AUC CGU UGU CCC GCC AUC UUG GCU CUC CUG GCG UUG GUU
met gln cys ile arg cys pro ala ile leu ala leu leu ala leu val
-111 -110                                      -100

    -90                                              -80
leu ser leu thr ala ser ile ser cys ser phe ala gly gln arg asp
CUG UCC CUG ACC GCC UCC AUC AGC UGC UCC UUC GCC GGA CAG AGA GAC
••• • •    •    •••  • •    •    •   ••• ••  •••
CUG UGC GGC CCA AGU GUU UCC UCC CAG CUC GAC AGA GAG CAG AGC GAC
leu cys gly pro ser val ser ser gln leu asp arg glu gln ser asp
                    -90                                      -80

                              -70
ser lys    leu arg leu leu leu his arg tyr pro leu gln gly ser
UCC AAA ::: CUC CGC CUG CUG CUG CAC CGG UAC CCG CUG CAG GGC UCC
 •   •     ••• • ••• • ••• •   • • ••   • ••• • •
AAC CAG GAC CUG GAC CUG GAG CUG CGU CAG CAC UGG CUG CUG GAG AGA
asn gln asp leu asp leu glu leu arg gln his trp leu leu glu arg
                                        -70

-60
lys                           gln asp met thr arg ser ala leu
AAA ::: ::: ::: ::: ::: ::: ::: CAG GAC AUG ACU CGC UCC GCC UUG
                                ••• ••   • • •        ••    ••
GCC CGG AGC GCC GGA CUC CUG UCC CAG GAG UGG AGU AAA CGG GCG GUG
ala arg ser ala gly leu leu ser gln glu trp ser lys arg ala val
            -60                                      -50

    -50                                              -40
ala glu leu leu leu ser asp leu leu gln gly glu asn glu ala leu
GCC GAG CUG CUC CUG UCG GAC CUC CUG CAG GGG GAG AAC GAG GCU CUG
•   ••• ••• ••        •     ••• •   • • •   •            •
GAG GAG CUG CUG GCU CAG AUG UCU CUG CCA GAG GCC ACG UUC CAG CGG
glu glu leu leu ala gln met ser leu pro glu ala thr phe gln arg
                        -40

                    -30                                      -20
glu glu glu asn phe pro leu ala glu gly gly pro glu asp ala his
GAG GAG GAG AAC UUC CCU CUG GCC GAA GGA GGA CCC GAG GAC GCC CAC
••• • • •••  •• ••      • • ••• •
GAG GCG GAG GAC GCG UCC AUG GCA ACA GAA GGA CGG ::: ::: ::: :::
glu ala glu asp ala ser met ala thr glu gly arg
    -30                                      -20

                              -10
ala asp leu glu arg ala ala ser gly gly pro leu leu ala pro arg
GCC GAC CUA GAG CGG GCC GCC AGC GGG GGG CCU CUG CUC GCC CCC CGG
    ••  ••• ••• ••• •• •     •             ••    •• •• ••
AUG AAC CUA GAG CGG UCC GUG GAC UCU ACC AAC AAC CUA CCC CCU CGU
met asn leu glu arg ser val asp ser thr asn asn leu pro pro arg
                                        -10
              Somatostatin I
              1                                    10
glu arg lys ala gly cys lys asn phe phe trp lys thr phe thr ser
GAG AGA AAG GCC GGC UGC AAG AAC UUC UUC UGG AAA ACC UUC ACC UCC
••• •  ••  ••  ••• ••  ••• ••• ••• •   ••• ••    • ••• ••  •••
GAG CGU AAA GCU GGC UGU AAG AAC UUC UAU UGG AAG GGC UUC ACU UCC
glu arg lys ala gly cys lys asn phe tyr trp lys gly phe thr ser
              1                                    10
              Somatostatin II
 14
cys OP
UGC UGA GCCGCUCCUCAUCCUCGCCGCUCCUCCGUCUCCAACGGACGUUUU:::::::ACAG
•• • •    ••••• •    •    ••• • ••••• ••   •      •    •••
UGU UAA ::AGCUCCGCCCAGCCAAAGCUACACCGUCACCGGACCAACCAAUCCCAGAUCAG
cys OC
 14
```

ACGCUGAAUGGAUCCCGGUUUGCAGCUCCUCCUUUUCUGGGCGGAGUCUGAAUGUAAACUUGA
•• ••••• •• •    • • •• •   •• •        ,• • ••• •• •
ACCCUGAAAUUCACCUGAAGAACUGGACCGACCAAUCAGCAGCUC:UCCGGAUGGAAUGUACC


UGAAACUAUUUUUAAUGGUUGGUUUGAAUAAAAAAUCUGUUUGAGAC  Poly A
•••• • •    •    •• • ••••• •••
UGAAUAAA:::::UAAUACUGUUAUGAAUUAAAGCGAUAAAUCCGUC  Poly A

**FIG. 3**